# EUROPEAN PATENT APPLICATION

(11) **EP 1 583 016 A2**
(43) Date of publication of application: **05.10.2005**
(21) Application number: 05004041.9
(22) Date of filing: 24.02.2005
(51) Int. Cl.: G06F 19/00

(54) **Data processing and display method for gene expression analysis system and gene expression analysing system**

(30) Priority: 30.03.2004 JP 2004100783
(71) Applicant: HITACHI SOFTWARE ENGINEERING CO., LTD., Kanagawa 230-0045 (JP)
(72) Inventor: Fujisaki, Ayoka c/o Hitachi Software Eng. Co. Ltd., Shinagawa-ku Tokyo 140-0002 (JP); Yamamoto,Noriyuki c/o Hitachi Software E. Co. Ltd., Shinagawa-ku Tokyo 140-0002 (JP); Mori, Atsushi c/o Hitachi Software Eng. Co. Ltd., Shinagawa-ku Tokyo 140-0002 (JP); Sakurai, Daisuke c/o Hitachi Software En. Co. Ltd., Shinagawa-ku Tokyo 140-0002 (JP); Yamashita,Iwao c/o Hitachi Software Eng. Co. Ltd., Shinagawa-ku Tokyo 140-0002 (JP); Kasuga, Takahiko c/o Hitachi Software En. Co. Ltd., Shinagawa-ku Tokyo 140-0002 (JP)
(74) Representative: Liesegang, Roland

(57) **Abstract**

In gene expression analysis using DNA chips, the position of a probe on a genome sequence, candidates for splice variants, and the like are displayed in relation to one another. Information on the position of the probe designed for a DNA chip system on the genome sequence is retrieved by connecting to an external database and a server that manage sequence information (genome sequence, mRNA, EST, etc.) and annotation information of various organisms. Further, homologous sequences of a gene for which the probe was designed are detected by homology search program, and the candidates for splice variants obtained from the search are stored in a DNA chip database in relation to expression data.

## Description

### FIELD OF THE INVENTION

The present invention relates to a gene expression analysis system using a DNA chip, and particularly to a system and a display method to display the position of a probe sequence on a genome sequence, candidates for splice variants, and translation products thereof.

### BACKGROUND OF THE INVENTION

In a gene expression analysis system, a DNA array or a DNA chip system (hereinafter, collectively referred to as DNA chip system) that allows expression levels of a number of genes to be determined at a time is used. Sequences of probes are designed from many gene coding regions of an organism, and these are mounted on the DNA chip system. To these probe sequences, mRNAs (target) isolated from the organism are hybridized. Gene expression levels can be determined by measuring the amounts of mRNAs that hybridize.

It becomes possible to predict a function of an unknown gene by studying expression levels of the gene in terms of time or tissue specific expression and further to map it in known metabolic pathways and interaction information. Prediction of regulatory relation of proteins that are gene products or of their functions is one objective of the DNA chip system.

In eukaryotes including human, mRNAs that hybridize to a probe contain not only mRNA that is translated into an objective gene product, but also mRNAs called splice variant and EST. The splice variants are formed by selective splicing, that is, specific exons are selected from among exon and intron sequences of a genome.

Although the splice variants are formed from a single gene region on a genome, these represent mutually different mRNAs. Accordingly, the splice variants are converted in vivo into proteins having mutually different sequences and perform mutually different functions. It is inferred that the total number of genes in human is about 35,000 and at least 35% of the genes undergo selective splicing (Non-patent Document 1).

Hence, it is considered that not only functions of proteins detected by a conventional DNA chip system but also novel proteins important in vivo can be discovered by monitoring effect of splice variants arising from selective splicing.

An example of a DNA chip system that allows splice variants to be monitored exhaustively is disclosed in Patent Document 1. In this DNA chip system, sequence specific expression levels are detected by measuring signal values on designed probes.

In Patent Document 2, a method to display exon and intron for splice variants is disclosed. In an exon-intron structure of a gene, the length of intron sequences is generally longer compared to the length of exons, and these sometimes differ by an order of magnitude or more. Therefore in the invention disclosed in Patent Document 2, the portions of intron sequences are displayed with compression in order to make the exon-intron sequences easily understandable, and mutually different portions in splice variants are displayed so as to be emphasized.

Further, information sites of public databases are available for acquisition of exon-intron display for splice variants. For example, there are UCSC (http://genome.ucc.edu/) and EMBLE (http://www.ensembl.org). When information concerning a specific gene sequence or a gene is input at these sites, homologous sequences to the input data such as genome sequence, mRNA, and EST are visually displayed. By virtue of this display, exon-intron sequences placed side by side with a genome sequence can be recognized.
[Non-patent Document 1] Lander, E.S., Linton, L.M. et al. (2001) Initial sequencing and analysis of the human genome. Nature, 409, 860-921
[Patent Document 1] JP-A No. 530894/2003 (P2003-530894A)
[Patent Document 2] JP-A No. 256434/2003 (P2003-256434A)

It is possible to detect expression levels of splice variants by the use of the DNA chip system disclosed in Patent Document 1. By designing exon-exon junction probes spanning over two exon sequences, expression levels of translation products in various combinations of two exons can be compared using a DNA chip having these probes. When the expression data obtained from the DNA chip system is analyzed, it is desirable that the expression data is exhaustively displayed so as to be related to portions of the gene used for designing the probes.

Although a reading device to measure hybridization intensities and software for their quantification are described in Patent Document 1, software to perform data mining is not disclosed. Even when the DNA chip designed in consideration of splice variants is used, a system that mutually recognizes information of the designed probes and the expression data is necessary during a process of the data mining.

Furthermore, Patent Document 2 and the information sites of public databases aim at displaying splice variants, and many splice variants for a specific gene are neither displayed at a time nor displayed in relation to their expression levels.

### SUMMARY OF THE INVENTION

Hence, the purpose of the present invention is to provide a gene expression analysis system using DNA chips and display method thereof in which a probe position on a genome sequence is displayed; candidates for splice variants (mRNA and EST sequences) hybridizing to the probe and their translation products are displayed; and expression data are displayed in relation to the probe.

To solve the above problems, the gene expression analysis system having the following functions is provided in the present invention:
1) A function to represent visually an expression pattern of genes on the entire chromosomes of an organism to be studied.
2) A function to represent visually where a probe sequence placed on DNA chips is positioned on the genome sequence and to display the sequence in relation to its expression level.
3) A function to display visually how splice variants and a probe sequence are located with respect to a genome sequence.
4) A function to display information on proteins that are the translation products of mRNAs of concern and to show whether there is any possibility that splice variants are translated to become proteins.
5) A function to connect to an external database that stores most recent genome information and detailed information (sequence and annotation information, etc.) on mRNAs and the like each downloaded from a plurality of public databases, and to a server that stores the BLAST (Basic Local Alignment Search Tool) search program.
6) A function to extract necessary information by the use of the external database and the server and to store it in a local database.

Namely, in a gene expression analysis system that analyzes a large volume of expression data obtained with a DNA chip system, the purpose of the present invention is to provide the gene expression analysis system loaded with a function in which numerical data representing expression values obtained from hybridization experiments and gene and sequence information concerning probes placed on DNA chips are input, and the numerical data are wholly corrected and preprocessed; a function in which the position of a probe on a genome sequence and candidates for splice variants (mRNA or EST sequences) that hybridize to the probe sequence are displayed; and a function in which a statistical analysis of the input numerical data is performed for classification and judgment according to statistical algorism.

The gene expression analysis system of the present invention is accessible to the information in the external database that manages sequence information and annotation information of various species of organisms in order to realize displaying the position of a probe on a genome sequence and the candidates for splice variants that hybridize to the probe sequence. Data exchange is performed via a network and an external server.

The external database stores detailed information such as accession number of each organism and gene name, and sequence data, and manages genome sequences in positional information of chromosomes.

In order to display the position of a probe on a genome sequence and the candidates for splice variants that hybridize to the probe sequence, functions that include the following five steps are provided in the present invention:

In the first step, information on chromosomes of an organism to be analyzed is extracted from the external database. That is, the number of chromosomes and lengths of long arm and short arm of each chromosome are retrieved and stored in a local DNA chip database.

In the second step, positional information of a probe sequence is extracted from gene information (mRNA, EST) by searching the external database using as a search key a probe annotation to obtain the positional information of the probe on the genome sequence based on the input numerical data, gene information concerning the probe, and the sequence information. The positions of probes on the genome sequence are determined by executing multiple alignments using all sequences of the probes on the DNA chip as query sequences and the genome sequence in the same position as the positional information of the probe as a subject sequence.

In the third step, candidates for splice variants (mRNA or EST sequences) that hybridize to the probe are extracted by searching the external database with the BLAST search program. The BLAST search program is executed by using the gene (mRNA) on the search key as a query sequence and all mRNA and EST sequences of the organism to be analyzed in the external database as subject sequences. Then, the BLAST search program is executed again by using each mRNA or EST obtained by the first BLAST search as a query sequence and the genome sequence of the organism to be analyzed in the external database as a subject sequence.

The BLAST search program is executed by the external server connected to the external database. The BLAST search program between base sequences is executed according to conditions of predetermined parameters using the entire sequence data of the databases to be searched as subject sequences. In this way, the position of a query sequence with respect to a subject sequence, and the position of the subject sequence with respect to the query sequence are determined. The BLAST search results are selected dependent on a condition and stored in the DNA chip database.

In the fourth step, a chromosome map is graphically depicted based on specified display data. A list of the initially specified display data is displayed in a tabular form (hybridization ID and DNA chip name). When a plurality of display data are selected by an input device, the selected data are displayed. Based on expression data of the selected DNA chip data, expression levels to display for each position on the genome are determined, and when plural data are selected, an average is calculated. When there exists a ratio (intensity) arising from a different probe ID with respect to the same position on the genome, an average value of ratios (intensity) for plural probe IDs is calculated and employed as an expression value for the position. A chromosomal map is graphically depicted based on the total number of chromosomes and the lengths of short arm and long arm of each chromosome, and the expression values are displayed on each position on the chromosomes in a way that a maximal value is in red, a median value in black, and a minimal value in green.

In the fifth step, the probe sequence and the splice variants are displayed such that their positional relations relative to the genome sequence are clearly understood. When a user selects a chromosome on the map with the input device, the selected chromosome number, the organism, and the display range of the genome sequence in an appropriate position are displayed. The sequence numbers of the range of the genome, the start number and the end number of the probe sequence are displayed. At the probe position, expression data are displayed as numerical values and in colors. Candidates for splice variants (mRNA and EST) are displayed at positions corresponding to the genome sequence based on the results from the BLAST search of each probe.

When a candidate for splice variant (mRNA and EST) is selected with the input device, detailed information on the selected sequence is displayed on a different window. When the probe ID is selected, the probe sequence, the annotation information on the gene that is input information, and the information on expression values are displayed. When the splice variant is selected, BLAST search result, the annotation information on the gene, and the sequence information are displayed.

According to the present invention, it becomes easy to obtain information on translation products of splice variants, since not only the position to relate expression data obtained by a probe sequence, that have been designed for a DNA chip system, to a genome sequence but also the candidates for splice variants (mRNA and EST) that hybridize to the designed probe sequence can be wholly observed.

Even when the analysis is performed with a DNA chip that has been designed in consideration of splice variants in advance, it becomes easy to understand visually in which position on the genome a probe sequence of interest was designed together with gene information.

An expression analysis using a DNA chip system to measure translation levels can be performed with higher accuracy by displaying information on proteins derived from splice variants and their expression values at the same time.

### BRIEF DESCRIPTION OF THE DRAWINGS

Fig. 1 is a structural diagram showing a gene expression analysis system of the present invention;
Fig. 2 is a schematic illustration showing an example of data obtained from an experiment carried out using a DNA chip;
Fig. 3 is a schematic illustration showing an example of a main screen after data input;
Fig. 4 is a schematic illustration showing an example of a data structure of a DNA chip database;
Fig. 5 is a schematic illustration showing an example of search result for a probe in the DNA chip database;
Fig. 6 is a flow diagram showing operations of programs stored in a program memory of the gene expression analysis system of the present invention;
Fig. 7 is a flow diagram showing a process of a splice variant display program;
Fig. 8 is a schematic illustration showing an example of a setting dialog to execute the splice variant display program;
Fig. 9 is a schematic illustration showing an example of a setting dialog to connect to an external database;
Fig. 10 is a schematic illustration showing a data structure of the external database;
Fig. 11 is a flow diagram showing a process of searching the external database for a probe position on a genome sequence;
Fig. 12 is a flow diagram showing a process of searching for splice variants;
Fig. 13 is a flow diagram of a process of executing the BLAST search program;
Fig. 14 is a flow diagram showing a process of executing a chromosome map display program;
Fig. 15 is a flow diagram showing a process of executing the splice variant display program;
Fig. 16 is a schematic illustration showing an example of display of a chromosome map and splice variants;
Fig. 17 is a schematic illustration showing an example of display of detailed information on a probe; and
Fig. 18 is a schematic illustration showing an example of display of detailed information on a splice variant.

### DETAILED DESCRIPTION OF THE PREFERRED EMBODIMENTS

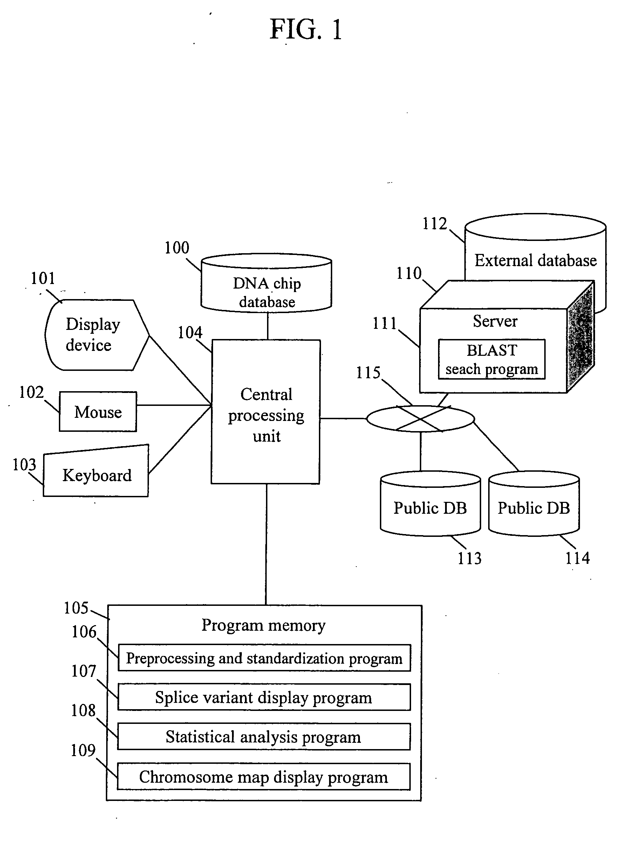

Hereinafter, an embodiment to carry out the present invention is specifically described with reference to the accompanying drawings. Fig. 1 illustrates a structure of a gene expression analysis system of the present invention. The gene expression analysis system of the present embodiment is provided with a DNA chip database 100, a display device 101, a mouse 102, a keyboard 103, a central processing unit 104, and a program memory 105. The gene expression analysis system is connected to a server 110, an external database 112, and further public (commercial) databases 113 and 114 via the Internet 115.

In the DNA chip database 100, information obtained from experiments carried out using a DNA chip system and information extracted from the external database 112 are stored. The display device 101 serves as an interface for data input and displays a chromosome map and splice variants both of which are objectives of the present invention. The mouse 102 and the keyboard 103 are used for selecting or inputting information by the user. The central processing unit 104 executes various programs and processing.

The program memory 105 is provided with a preprocessing and normalization program 106 to convert input expression data into values of significance or correct them in advance, a splice variant display program 107 to display genomic sequences, probe sequences, and mapping of splice variants, a statistical analysis program 108 to perform a statistical analysis necessary for a gene expression analysis, and a chromosome map display program 109 to display chromosomes together with expression levels.

It should be noted that these pieces of software may be replaced with pieces of hardware. That is, a preprocessing and normalization unit, a splice variant display unit, a data processing unit, and a chromosome map display unit may be provided in place of the preprocessing and normalization program 106, the splice variant display program 107, the statistical analysis program 108, and the chromosome map display program 109.

The server 110 to process the external database 112 and the external database information is connected to a plurality of the public (commercial) databases 113 and 114 via the Internet 115. Accordingly, the external database 112 and the server 110 download data periodically from the public (commercial) databases 113 and 114 and prepare indexes from its character string, thereby keeping databases in each index.

The server 110 is provided with the BLAST search program 111. The BLAST search program 111 can be used in the gene expression analysis system of the present invention. When a specific base sequence or amino acid sequence is input as a query sequence using the input devices 102 and 103, sequences similar to the input sequence are searched in the external database 112.

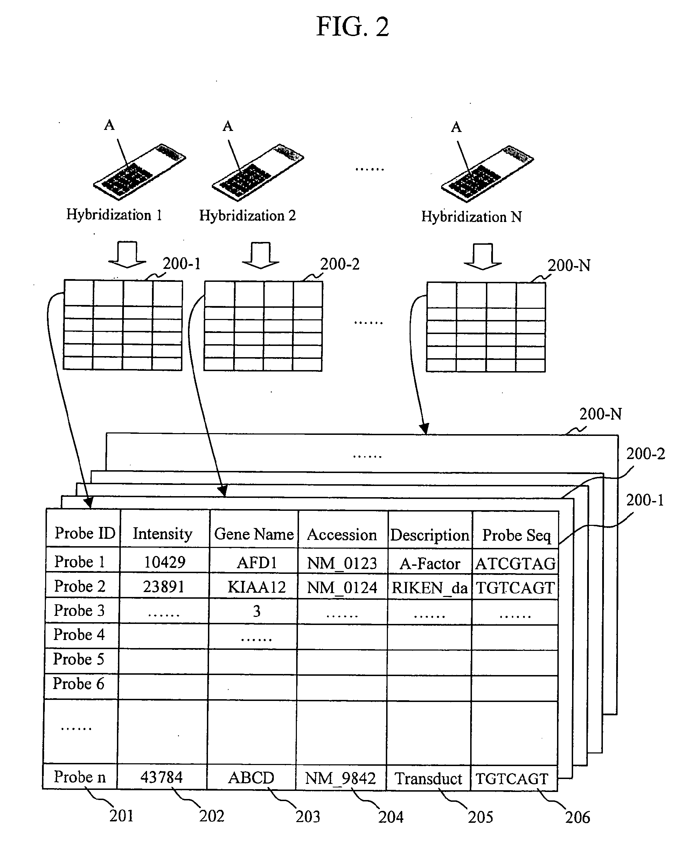

Fig. 2 illustrates an example of an experiment carried out using a DNA chip. A DNA chip A carrying DNA probes of an organism to be studied is prepared. mRNAs obtained from a study sample are converted into cDNAs, labeled with a fluorophore, and hybridized to the DNA probes on the DNA chip A. The fluorescence intensity of the DNA probes after the hybridization reaction is measured. As the result of the hybridization in one experiment on the DNA chip A, a piece of experimental data 200-1 is obtained. As the results of the hybridization in N experiments, N pieces of experimental data, 200-1 to 200-N, are obtained. The experimental data 200-1 to 200-N thus obtained are used for analysis using the gene expression analysis system of the present invention.

The experimental data 200-1 obtained from hybridization includes probe identification numbers 201, fluorescence intensities on each probe 202, gene names of genes hybridized to each probe 203, accession numbers to the public databases 204, descriptions 205, and probe sequences 206 as illustrated. Information related to the probes or the genes other than those described may also be included. The fluorescence intensity 202 is of one kind or of two kinds. When the number of target DNA samples to be subjected to hybridization is two in kinds, two kinds of fluorescent dyes are sometimes used.

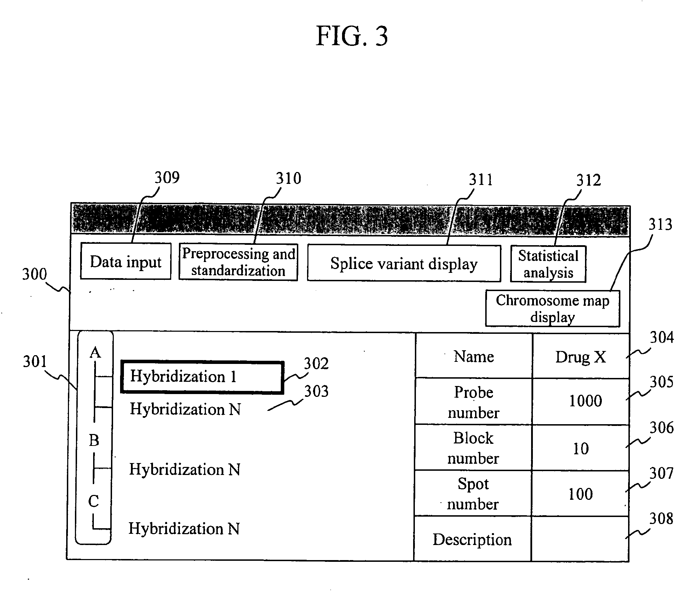

Fig. 3 illustrates an example of a main screen 300 to be displayed on the display device of the gene expression analysis system of the present invention. It is assumed that the N pieces of experimental data, 200-1 to 200-N, shown in Fig. 2 are input as input data. The input data may be input from a storage medium such as hard disk or CD-ROM via a program of the gene expression analysis system. The input method includes input from a menu, input by drag and drop, and the like.

The input data is displayed in a tree structure 301, and the kinds of DNA chips A, B, and C are shown in the first column. For each DNA chip, the number of experiments, i.e. the number of hybridization is displayed. For example, in the case of the DNA chip A, the number of hybridization is displayed in N as shown by reference numerals 302 and 303. When the user selects, for example, hybridization 1 as shown by the reference numeral 302 with the input devices 102 and 103, the experimental data of the hybridization 1 is displayed on the right view. In the illustrated example, an experiment name 304 to represent hybridization uniquely such as the name of sample subjected to each hybridization, a total probe number 305 on the DNA chip, a total block number 306 on the DNA chip, a spot number 307 in one block, and other detailed description 308 are displayed.

In the upper part of the screen 300, a tool button 309 to implement wizard for data input, a tool button 310 to execute the preprocessing and normalization program 106, a tool button 311 to execute the splice variant display program 107, a tool button 312 to execute the statistical analysis program 108, and a tool button 313 to execute the chromosome map display program 109 are provided. The user can perform desired processes by clicking on these buttons.

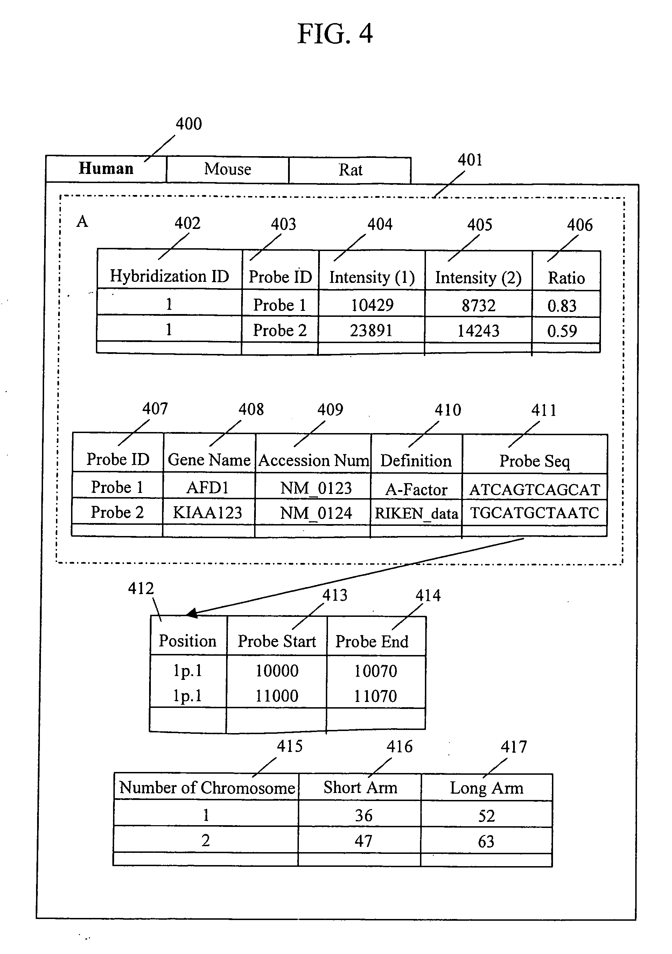

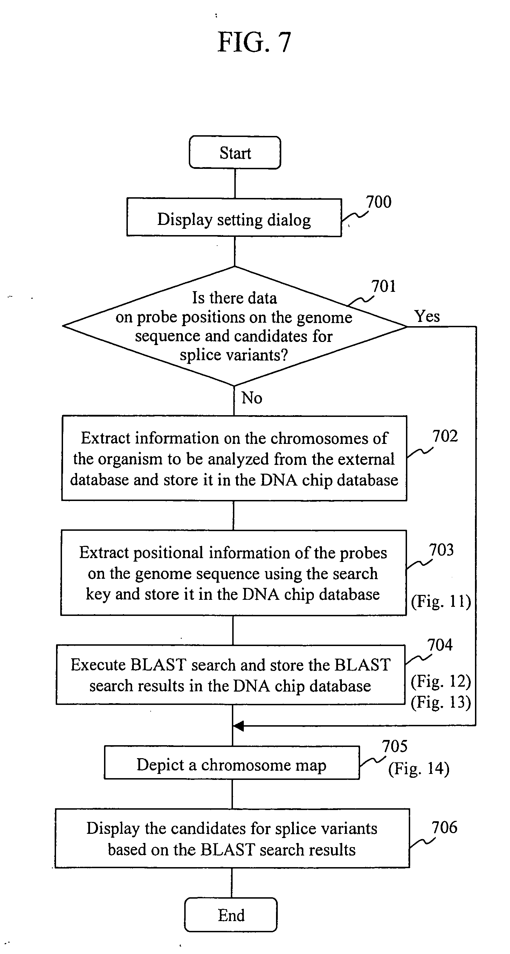

Fig. 4 illustrates a data structure inside the DNA chip database 100. In the DNA chip database 100, data is managed for every species of organisms. Thus, a category 400 representing species of organisms such as human, mouse, and rat is included. Although three kinds of species of organisms are shown in the example, the category should be provided for all species of organisms. The data stored in the DNA chip database 100 includes experimental data 401 input by users and data 412 to 417 extracted from the external database 112. The method to extract data from the external database 112 is explained later with reference to Fig.7.

Since the target of the DNA chip A is human (Homo sapiens), the experimental data 401 obtained with the DNA chip A is stored under the category of human. As illustrated, the experimental data 401 includes a hybridization ID 402 to represent each hybridization uniquely, a probe ID 403 in each hybridization, expression data (fluorescence intensities) 404 and 405, and an expression ratio 406. There exist two cases for the expression data that are one kind and two kinds. In the case of two kinds, the expression ratio 406 is indicated.

The expression ratio 406 is determined by Intensity (1)/Intensity (2) or Intensity (2)/Intensity (1).

The experimental data 401 includes further a gene name 408 of the gene that hybridizes to a DNA sequence serving as a probe, an accession number 409, a definition of the gene 410, and the probe sequence 411.

The data 412 to 417 extracted from the external database 112 includes positions 412 of the probes representing chromosomal positions on the genome sequence, starting positions 413 and ending positions 414 of the probe sequences on the genome sequence, the number of chromosomes 415 in the organism, and the lengths of short arm 416 and long arm 417 of the chromosomes.

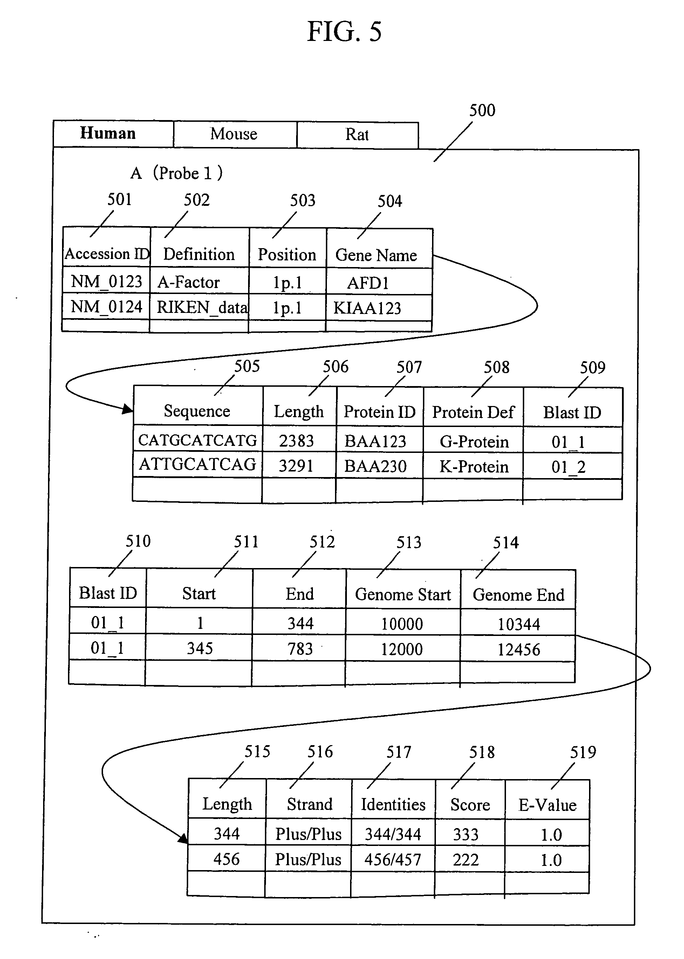

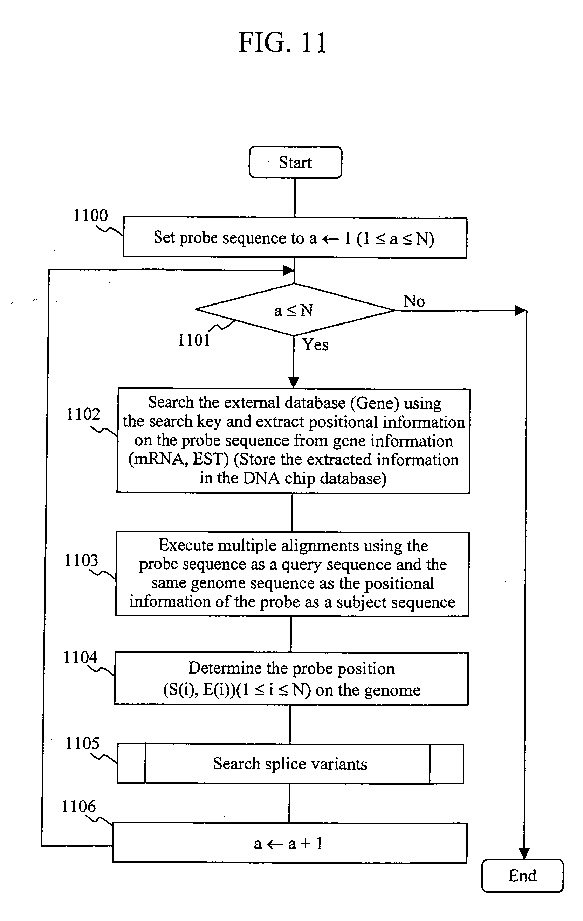

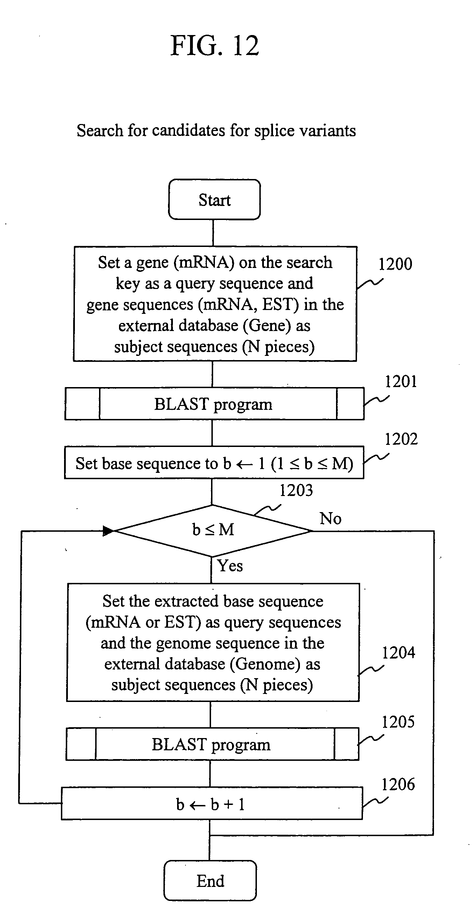

Fig. 5 illustrates candidates for splice variants (mRNA and EST sequences) that hybridize to the probe. The candidates for splice variants are retrieved from the external database 112 with the BLAST search program 111 provided in the server 110. It should be noted that the search of the candidates for splice variants is carried out when the splice variant display program 107 is executed. The detailed search process of the candidates for splice variants is shown in the flow charts of Figs. 11 and 12.

The user searches for gene sequences (mRNA sequences) using the gene name 408 or the accession number 409 as a search key among the input experimental data 401. When sequence information is found, BLAST search is performed in mRNA and EST sequence databases using the found sequence as a query sequence. As the result, it is assumed that genes similar to the gene on the search key are found. Of each gene, an accession ID 501 to protein database, a definition 502, a probe position 503 on the genome sequence, a gene name 504, a base sequence 505, a length of the base sequence 506, a protein ID 507, and a protein definition 508 are obtained and stored.

Then, where each gene is located on the genome is determined by BLAST search using the base sequence 505 as a query sequence. When BLAST search IDs 509 and 510 are obtained as the result of the BLAST search, these are stored. A gene sequence is derived from an exon-intron structure on the genome, and the exon sequences are scattered. For this reason, a start position 511 and an end position 512 of each fragment of the gene sequence, and a start position 513 and an end position 514 corresponding to these respective positions on the genome sequence are stored. Further, a length of sequence in the exon portion 515, an orientation of genome sequence (strand) 516, identities to the genome sequence 517, a score of BLAST search 518, and an E-value 519 are stored.

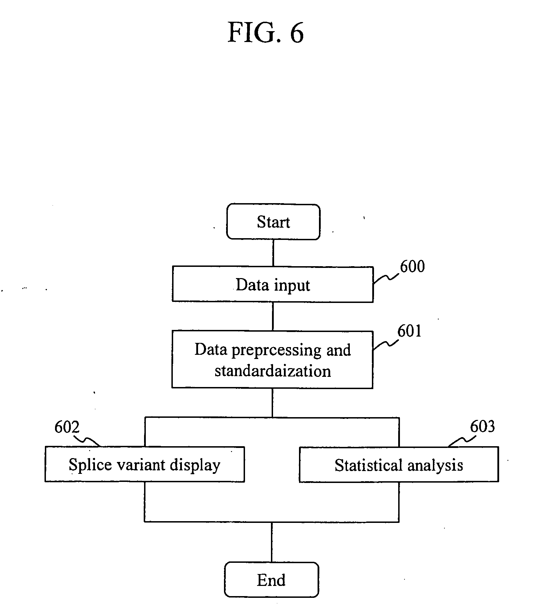

Fig. 6 illustrates a flow of operations of programs stored in the program memory 105 of the gene expression analysis system of the present invention. In step 600, the user inputs data from hybridization experiments with DNA chips. By clicking the tool button 309 on the main screen 300 shown in Fig. 3, the data input wizard is set in motion. The user inputs the experimental data according to the data input wizard.

In step 601, preprocessing and normalization are carried out by the preprocessing and normalization program 106. By clicking on the tool button 310 on the main screen 300 shown in Fig. 3, the preprocessing and normalization program is executed. The preprocessing represents execution of judging whether the input expression data is right or wrong, correcting them if necessary, and narrowing down data to be used for analysis based on criteria of judgment for each spot on the DNA chip. When experiments such as reproducibility experiments using a plurality of the same DNA chips, experiments to exchange two different fluorescent dyes, and experiments to confirm reliability of data are carried out, it is possible to extract highly reliable data from these experimental data.

The normalization represents converting data having a linear or non-linear distribution into data having a normal distribution for the whole analysis data on each DNA chip. By performing the normalization, all of the extracted data are made possible to be compared.

After completing the preprocessing and normalization by the preprocessing and normalization program 106, the user selects either the splice variant display program 107 or the statistical analysis program 108. Clicking on the tool button 311 on the main screen 300 allows to proceed to step 602, and the splice variant display program 107 is executed, while clicking on the tool button 312 allows to proceed to step 603, and the statistical analysis program 108 is executed. The details of the splice variant display program 107 and the statistical analysis program 108 are explained below.

An outline of a processing flow of the splice variant display program 107 is explained with reference to Fig.7. The splice variant display program 107 is executed by clicking on the tool button 311 on the main screen 300 shown in Fig. 3.

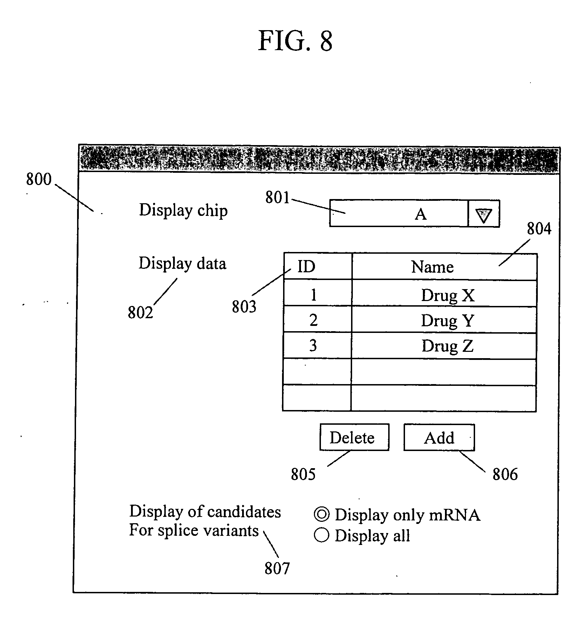

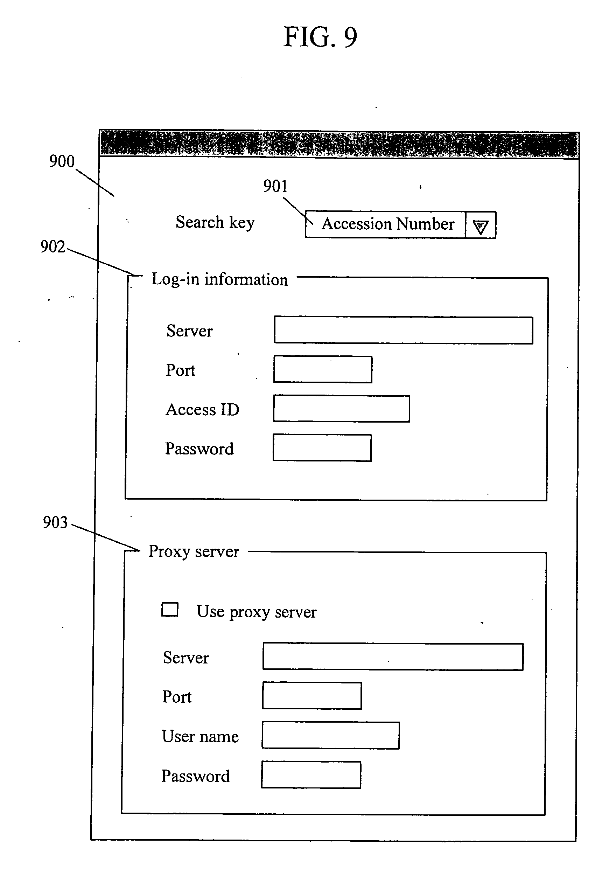

At step 700, a setting dialog is displayed on the screen of the display device 101. An example of the setting dialog is explained later with reference to Figs. 8 and 9. The splice variant display program 107 is executed according to the setting displayed on this setting dialog.

In step 701, the user judges whether or not data on the probe positions of the DNA chip A to be displayed on the genome sequence and data on candidates for splice variants are stored in the DNA chip database 100. In other words, the user judges whether or not the information 412 to 414 and 501 to 519 concerning the DNA chip A has already been extracted from the external database 112. When there are the data on the positions of the probes of the DNA chip A on the genome sequence and the data on candidates for splice variants, these are supposed to be displayed in appropriate boxes in the setting dialog shown in Figs. 8 and 9. When the information 412 to 414 and 501 to 519 concerning the DNA chip A has already been extracted from the external database 112, the external database 112 is not repeatedly searched. Accordingly, the splice variant display program 107 proceeds to step 704 and carries out only image creation process.

When the information 412 to 414 and 501 to 519 concerning the DNA chip A has not yet been extracted from the external database 112, the splice variant display program 107 proceeds to step 702.

In the step 702, information such as the number of chromosomes 415 of the organism, the lengths of short arms 416, and the lengths of long arms 417 is obtained from information 1013 to 1015 of the external database 112. In step 703, the probe positions 412 on the genome sequence and the starting positions 413 and ending positions 414 of the probes on the genome sequence are obtained using the search key. The details of the step 703 are explained with reference to Fig. 11.

In step 704, candidates for splice variants are searched by the BLAST search program, and the positions 513 and 514 of splice variants on the genome sequence are obtained. The results of the BLAST search are stored in the DNA chip database 100. Based on the data 400 stored in the DNA chip database 100 shown in Fig. 4 and the candidates of splice variants obtained from the external database 112 shown in Fig. 5, an image of chromosome map is created in step 705. Finally in step 706, the candidates for the splice variants are displayed so as to be arranged on the specified genome sequence on the basis of the BLAST search results.

Fig. 8 illustrates an example of a setting dialog 800 of a display chip displayed on the screen of the display device 101 when the splice variant display program 107 is executed. This setting dialog 800 includes a display DNA chip 801, display data 802, a hybridization ID 803, an experiment name 804, a delete key 805, an add key 806, display of candidates for splice variants 807, and the like.

The display DNA chip 801 is displayed in a pull-down menu, and the kinds of the DNA chips 301 in Fig. 3 are displayed in the pull-down menu. When the display DNA chip 801 is designated, respective items 803 and 804 of the display data 802 are displayed. Since the DNA chip A is designated in the illustrated example, the hybridization IDs 803 of hybridization experiments carried out using the DNA chip A and the experiment names 804 are displayed. When it is necessary to delete the displayed hybridization IDs 803, the delete key 805 is used, and when it is necessary to add, the add key 806 is used. What can be added is a hybridization ID registered in relation to the DNA chip A. The display of candidates for splice variants 807 is either one of "display only mRNA" or "display all (short fragments such as EST included)".

Fig. 9 illustrates an example of a search key setting dialog 900 that is displayed on the screen of the display device 101 when connected to the external database 112. The setting dialog 900 includes a search key 901, and an address of server, a port number, an access ID, and a password 902 that are needed to connect to the external database 112 and the server 110. When a proxy server is used to connect to the server, setting 903 is taken into consideration. Only when the proxy server is utilized, the check box is checked on so that the address of server, the port number, a user name, and a password can be set.

The search key 901 is displayed in a pull-down menu, and the gene name 408 or the accession number 409 in Fig. 4 is displayed in the pull-down menu. That is, the user selects the gene name 408 or the accession number 409 for the search key.

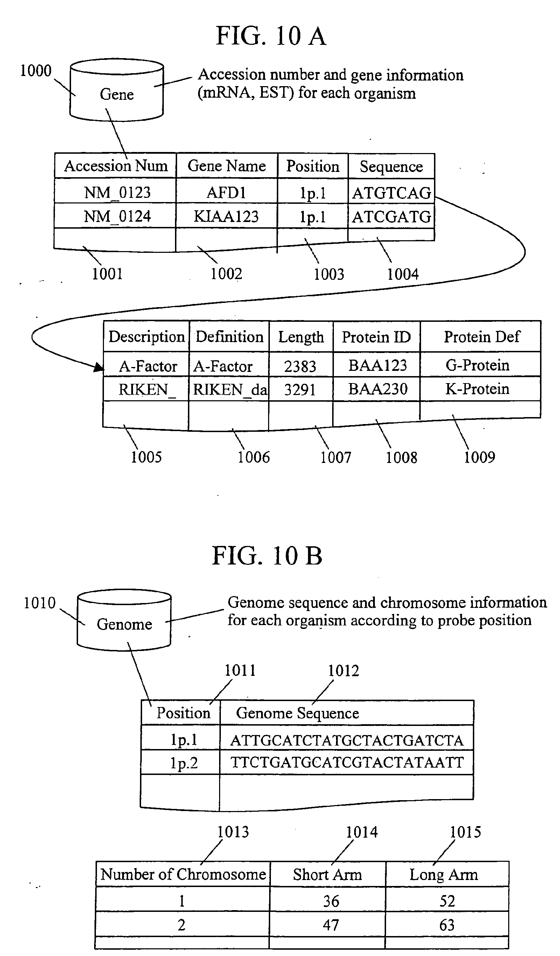

Fig. 10 illustrates an example of an internal structure of the external database 112. As described above, the external database 112 downloads periodically data from a plurality of the public databases 113 and 114 storing biological information that are connected via a network. Representative public databases include an example below.

GeneBank (1080623142687_0): NCBI (National Center for Biotechnology Information)

The external database 112 is provided with, for example, a first data table 1000 that stores accession numbers and gene data for each species of organisms. An index search of this data table 1000 is possible by the use of an accession number. This data table 1000 includes accession numbers 1001, gene names 1002, positions of probes on the genome sequence 1003, gene sequences 1004, descriptions 1005, definitions 1006, lengths of gene sequences 1007, link IDs (protein IDs) 1008 to databases of proteins that are gene products, and descriptions 1009 of proteins.

The external database 112 is provided with, for example, a second data table 1010 that stores probe positions on genome sequences and genome sequence data for each species of organisms. An index search of this data table 1010 is possible by the use of a probe position on a genome sequence. In this data table 1010, positions on chromosomes of genome sequence 1011, genome sequence 1012, numbers of chromosomes 1013, lengths of short arms 1014, and lengths of long arms 1015 of the chromosomes are stored.

The details of the step 703 in Fig. 7 are explained with reference to Fig. 11. As described above, whether or not the information 412 to 414 and 501 to 519 concerning the DNA chip A is stored in the DNA chip database 100 is judged before executing the splice variant display program. When the information has not been stored, it is extracted from the external database 112. Accordingly, when the information 412 to 414 and 501 to 519 concerning the DNA chip A has already been stored in the DNA chip database 100, the process of the steps 702 to 704 in Fig. 7 is not performed.

Step 1100, step 1101, and step 1106 indicate conditions under which a search process is performed for all probes from the first to the Nth probe sequences. The search process from steps 1102 to 1105 is performed for each probe. The contents of the steps from 1102 to 1105 are explained below.

In the step 1102, the first data table 1000 in the external database 112 is searched using the search key 408 or 409 and the probe sequence 411. When the probe position 1003 on the genome sequence is obtained, it is stored in the probe position 412 on the genome sequence in Fig. 4. For example, it is assumed that the accession number 409 is chosen as a search key. When the same number as that of the search key is present in a column of the accession number 1001 in Fig. 10, the probe position 1003 in the same row is stored in the probe position 412 on the genome sequence in Fig. 4.

In step 1103, the genome sequence in the same position as the probe position 412 is searched from the second data table 1010 of the external data base 112 by using the probe sequence 411 as a query sequence. When an appropriate genome sequence is found, multiple alignments are performed with the found sequence as a subject sequence. The probe position on the genome sequence (S(i), E(i)) (1≤i≤N) is determined in step 1104. S(i) is stored in the start position 413 of the probe sequence on the genome sequence in Fig. 4, and E(i) is stored in the end position 414 in Fig. 4. Then, splice variant search is carried out in step 1105. The details are explained with reference to Fig. 12.

The step 704 in Fig. 7 and the step 1105 in Fig. 11 are explained with reference to Fig. 12. In step 1200, a search key and a search subject sequence are set. For example, when the accession number 409 is set for the search key, the column of the accession numbers 1001 of the first data table 1000 in the external database 112 shown in Fig. 10 is searched, and the gene sequence 1004 in the row corresponding to the appropriate accession number is set as a query sequence. When the gene name 408 is set for the search key, the column of the gene names 1002 of the first data table 1000 in the external database 112 shown in Fig. 10 is searched, and the gene sequence 1004 in the row corresponding to the appropriate gene name is set as a query sequence. All of the gene sequences 1004 registered in the first data table 1000 of the external data base 112 become subject sequences.

In step 1201, BLAST search is performed for all of the gene sequences that become subject sequences, and the candidates for splice variants that hybridize to the probe are searched.

Steps 1202, 1203, and 1206 indicates conditions under which M pieces of acceptable base sequences that meet the conditions are extracted from the BLAST search results and all of the extracted base sequences from the first to the M-th pieces are subjected to search process without exception. The search process from the step 1204 to 1205 is performed for each base sequence. The process contents of the steps 1204 and 1205 are described below.

In the step 1204, the detailed information on the M pieces of the base sequences are first obtained from the items 1001 to 1009 of the first data table 1000 in the external database 112 shown in Fig. 10, and these are stored in the items 501 to 508 in Fig. 5. The M pieces of the base sequences to be processed are used as query sequences. All genome sequences of the second data table 1010 in the external database 112 are used as subject sequences. In the step 1205, BLAST search is performed.

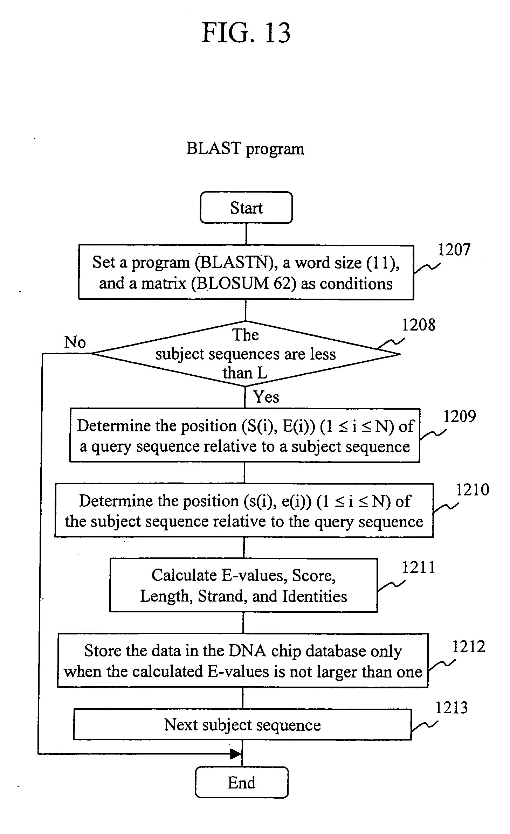

The process of the BLAST search program in the steps 1201 and 1205 is explained with reference to Fig. 13. At step 1207, conditions for the program are set. The program to be executed is BLASTN, the word size is 11, and the matrix is BLOSUM 62. However, these conditions are allowed to be varied. The step 1208 shows the condition under which the results of the BLAST search are registered in the DNA chip database 100. In this instance, when subject sequences registered in the DNA chip database 100 are M pieces and when effective results are obtained by examining the results from L pieces of the subject sequences, the subject sequences are registered in the DNA chip database 100.

In step 1209, the BLAST search program is executed, and the positions of a query sequence relative to the subject sequence (S(i), E(i)) (1≤i≤N) are determined. In step 1210, the positions of the subject sequence relative to the query sequence (s(i), e(i)) (1≤i≤N) are determined. In step 1211, E-Value and Score are calculated, and Length, Strand, and Identities are obtained. Only when the calculated E-Value is not larger than one, the information is stored in the DNA chip database 100 in step 1212. In step 1213, a BLAST search ID 509 is given to the BLAST search results, and the search results are stored in the items 511 to 519 in Fig. 5. S(i) is stored in the start position 511 of the gene sequence, E(i) in the end position 512 of the gene sequence, s(i) in the start position 513 of the genome sequence, and e(i) in the end position 514 of the genome sequence. Then, the next subject sequence is examined.

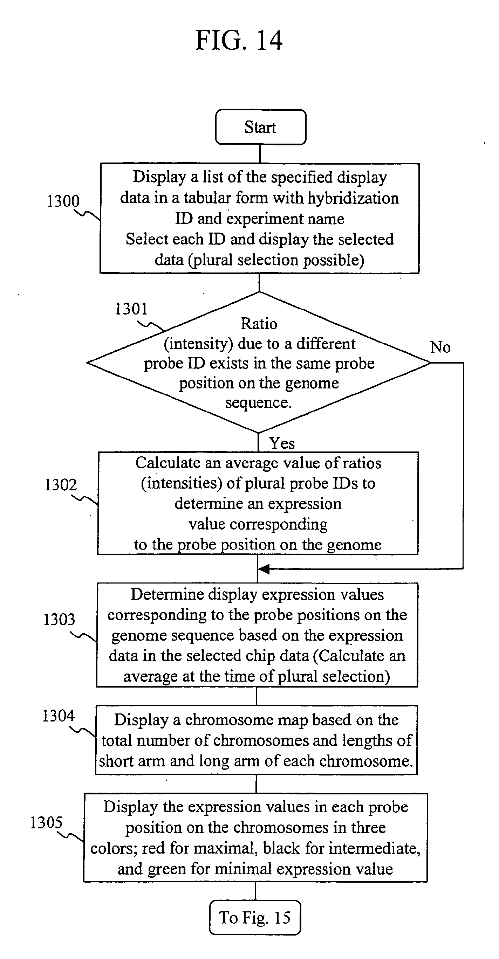

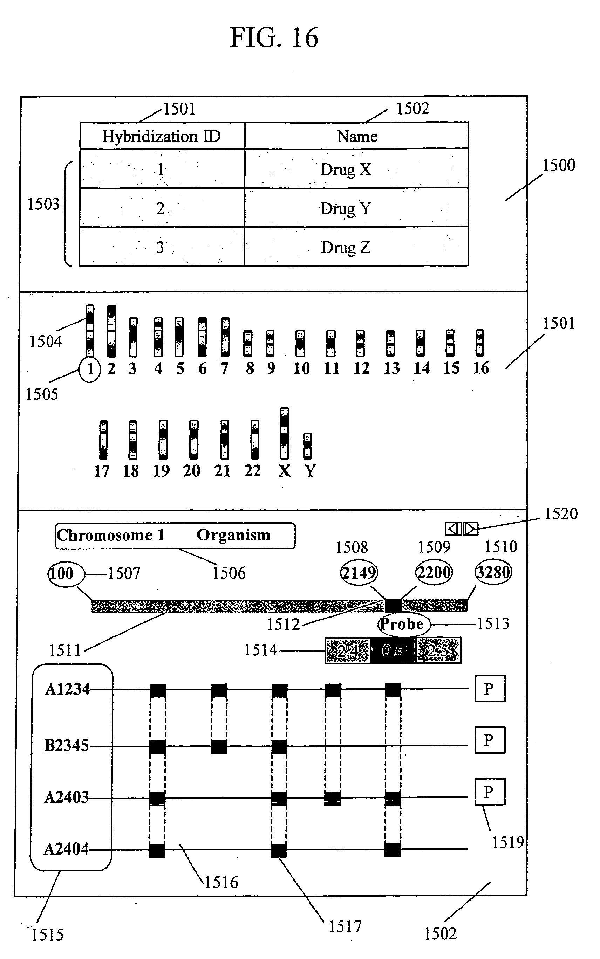

Fig. 14 represents a process flow of the step 705 in Fig. 7. The step 705 is executed by the chromosome map display program 109. First, the name of the data (hybridization ID and experiment name) designated as display data is displayed in a tabular form in step 1300. That is, a list 1500 of the display data in Fig. 16 is displayed. When the user selects a data name with the input devices 102 and 103, the selected data 1501 and 1502 are displayed. It is possible to select a plurality of data.

In step 1301, whether or not a different probe ID for the same probe position on the genome sequence exists is judged. When there is a different probe ID, the process is advanced to step 1302, and an average value of ratios (intensity) of plural probe IDs is determined as an expression value of the probe position.

When there is no different probe ID, the process is advanced to step 1303, and the expression level corresponding to the probe position is determined on the basis of expression data values of the selected data. When plural data are selected in the step 1300, an average of expression levels of the probe position of each DNA chip is taken as the expression level.

In step 1304, a chromosomal map is displayed based on the total number of chromosomes 415, the lengths of short arm of chromosomes 416, and the lengths of long arm of chromosomes 417. Finally, expression levels are displayed in the probe positions on each chromosome in step 1305. Expression levels are expressed in three colored levels; red for maximal expression value, black for median expression value, and green for minimal expression value. In this way, a chromosomal map 1501 in Fig. 16 can be depicted graphically.

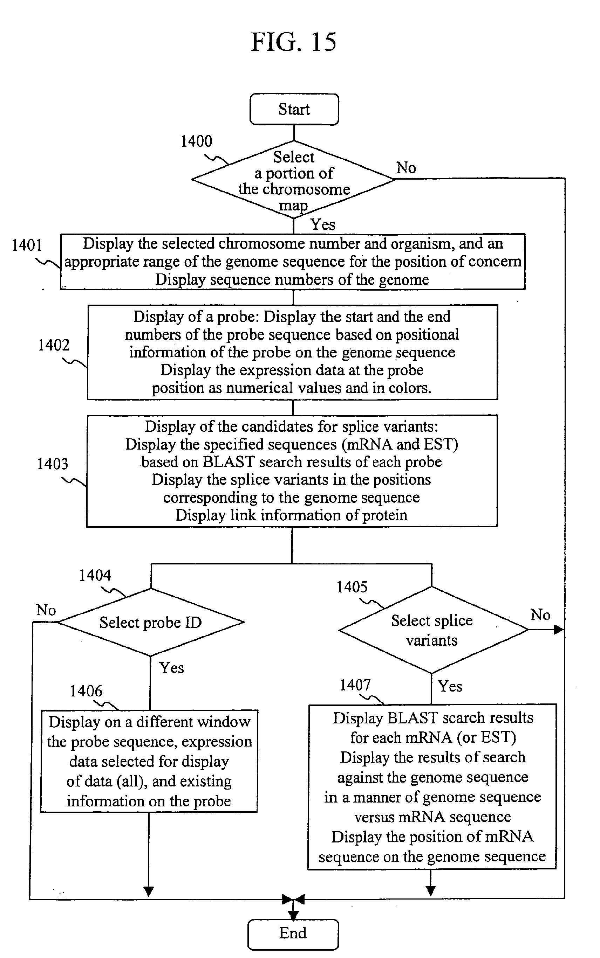

Fig. 15 represents a process flow of the step 706 in Fig. 7. The step 705 is executed by the splice variant display program 107. Here, it is assumed that the screen shown in Fig. 16 has been displayed on the display device 101 as a premise of executing the splice variant display program 107. Accordingly, Fig. 16 is referred to when needed.

In step 1400, the user clicks on a chromosome 1504 in the chromosome map 1501 with the input devices 102 and 103, and makes it selected. By this means, an operation of the splice variant display program 107 is started. In step 1401, the chromosome number and organism 1506 are displayed, and the genome sequence 1511 in a certain length of the genome is displayed in a strip shape. Further, sequence numbers 1507 and 1510 of the genome sequence are displayed to specify clearly the display range of the genome.

In step 1402, the probe position on the genome sequence is displayed. The start position 1508 and the end position 1509 on the sequence corresponding to the probe position are displayed. A probe ID 1513 is displayed at the probe position, and expression data 1514 is displayed as numerical values and in colors.

In step 1403, the candidates for splice variants that hybridize to the probe are displayed. Based on BLAST search results of the probe, only the specified sequences (mRNA and EST) 1515 are displayed. Exon portions 1517 and intron portions 1516 of each sequence are displayed such that the positions of the splice variants corresponding to the genome sequence can be seen. In addition, when there is information on protein that is the translation product of the gene in protein databases, link information 1519 is displayed.

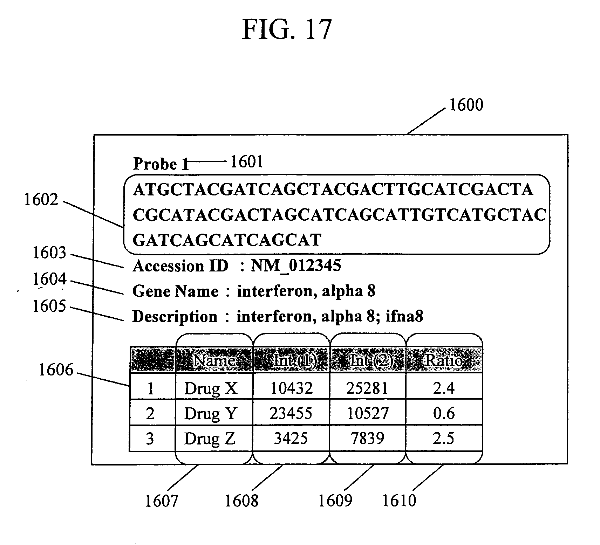

When the user selects the probe position 1512 or the probe ID 1513 on the screen shown in Fig. 16 in step 1404, the detailed information 1600 on the probe shown in Fig. 17 is displayed on a different window in step 1406.

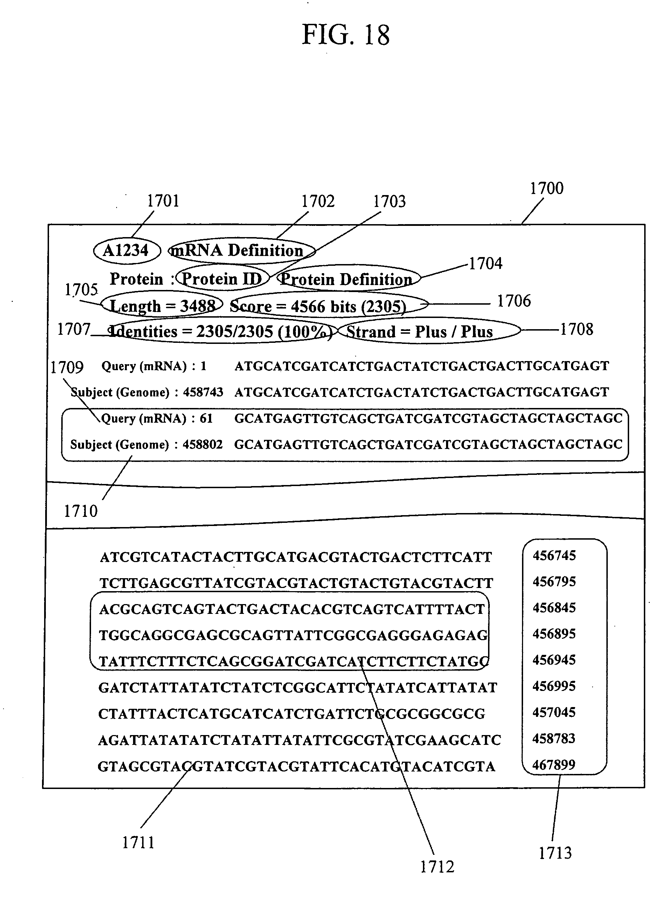

In step 1405, when the user selects the sequence (mRNA or EST) 1515 in the splice variant display 1520 on the screen shown in Fig. 16, the detailed information 1700 on the splice variant shown in Fig. 18 is displayed on a different window in step 1407.

Fig. 16 represents a list of the display data 1500, a chromosome map 1510 and the splice variant display 1520 which were obtained by the process in the steps 704 and 705 in Fig. 7. The list of the display data 1500 and the chromosome map 1510 are displayed when the process in Fig. 14 is performed. The list of the display data 1500 displays the hybridization ID 1501 and the experiment name 1502 that have been predetermined by the user on the display chip 801 and the display data 802 in the setting dialog 800 shown in Fig 8. In the example illustrated, three hybridization IDs 1503 are selected and displayed.

In the chromosome map 1510, the chromosomes 1504 and chromosome numbers 1505 are displayed. Since the number of chromosomes and the lengths of short arm and long arm differ depending on species of organisms, the chromosomes 1504 are depicted graphically based on the information 415 to 417 of an organism. Expression levels in each position on the genome are calculated from the process in Fig 14 using the DNA chip data selected on the list of display data 1500. The expression levels are graphically displayed in colors; red for maximum values, black for median values, and green for minimum values.

The splice variant display 1520 displays the chromosome selected from the chromosome map 1510. The chromosome number and organism 1506, and the genome sequence 1511 in a certain genome length are displayed in a strip shape. The sequence numbers (100) 1507 and (3280) 1510 of the genome sequence to indicate the display range of the genome are displayed.

The probe position 1512 on the genome sequence is displayed, and the start number 1508 and the end number 1509 on the sequence are displayed. At the probe position 1512, the probe ID 1513 and the expression data 1514 are displayed. The expression data 1514 consists of expression ratios and color display to represent their magnitudes. The color display is shown in red for a maximum value, in black for a median value, and in green for a minimum value. In the present example, three data have been selected on the list of display data 1500, and therefore three kinds of expression data are displayed. Each value represents an expression ratio and displayed in color according to the magnitude of the expression ratios, where the maximum expression value is in red, the median value in black, and the minimum value in green.

The splice variant display 1520 includes the specified sequences (mRNA and EST) 1515 obtained from BLAST search results of each probe. The exon portions 1517 and the intron portions 1516 for the sequences of each splice variant are displayed so as to correspond to the genome sequence 1511. When there is information on protein, the translation product of the gene, in the protein database of the external database 112, the link information 1519 is displayed. The length of the genome sequence displayed on one screen can be adjusted. Further, the display portion of the screen can be shifted toward the right direction or the left direction by clicking on the go button 1520.

An example of the screen to display detailed information of a probe is explained with reference to Fig. 17. A screen 1600 is displayed by clicking on the probe position 1512 or the probe ID 1513 on the splice variant display 1502 in Fig. 16. This screen 1600 includes a probe ID 1601, a probe sequence 1602, an accession number 1603, a gene name 1604, and a description 1605. These pieces of information can be obtained from the input data stored in the items 407 to 411 shown in Fig. 4. Further, expression data 1606 of the selected data is displayed in a tabular form. The expression data 1606 includes an experiment name 1607 of the selected data, expression values 1608 and 1609, and an expression ratio 1610. The expression data 1606 is obtained from the input data stored in the items 404 to 406 shown in Fig. 4.

An example of a screen to display the results of BLAST search for candidates for splice variants (mRNA or EST sequence) is explained with reference to Fig. 18. A screen 1700 is displayed by clicking on the sequence (mRNA or EST sequence) 1515 on the splice variant display 1502 in Fig. 16. Here, a case in which the candidate for splice variant is mRNA is explained. Basic information such as an accession number 1701, a definition of mRNA 1702, a protein ID 1703, and a protein definition 1704 is displayed. These pieces of information are obtained from the data stored in the items 501, 502, 507, and 508 of the DNA chip database 100 shown in Fig. 5.

Based on the basic information, a BLAST search result is displayed. That is, a sequence length 1705 of the query sequence (mRNA in this case), a score 1706, identities 1707 which represent how much the subject sequence has matched the query sequence, and strand 1708 that is the direction of the subject sequence with respect to the query sequence are displayed. These data are obtained from the data stored in the items 515 to 518 of the DNA chip database 100 shown in Fig. 5.

The results 1709 and 1710 that represent how much the mRNA sequence and the genome sequence match each other are displayed. These results 1709 and 1710 are obtained from the data stored in the items 511 to 514 of the DNA chip database 100 shown in Fig. 5. By aligning two sequences with each other in this way, the degree of matching can be visually viewed. This display method is similar to that of BLAST search result of NCBI. Further, an exon sequence (mRNA fragment) 1712 in a genome sequence 1711 is displayed. The exon portion is displayed by half-tone dot meshing of a portion of the genome sequence. Furthermore, sequence numbers 1713 of the genome sequence are displayed.

In the foregoing, the embodiment of the present invention has been explained. However, the present invention is not limited to the above embodiment, and it should be understood by one of ordinary skill in the art that various modifications can be made without departing from the scope of the invention set forth in the appended claims.

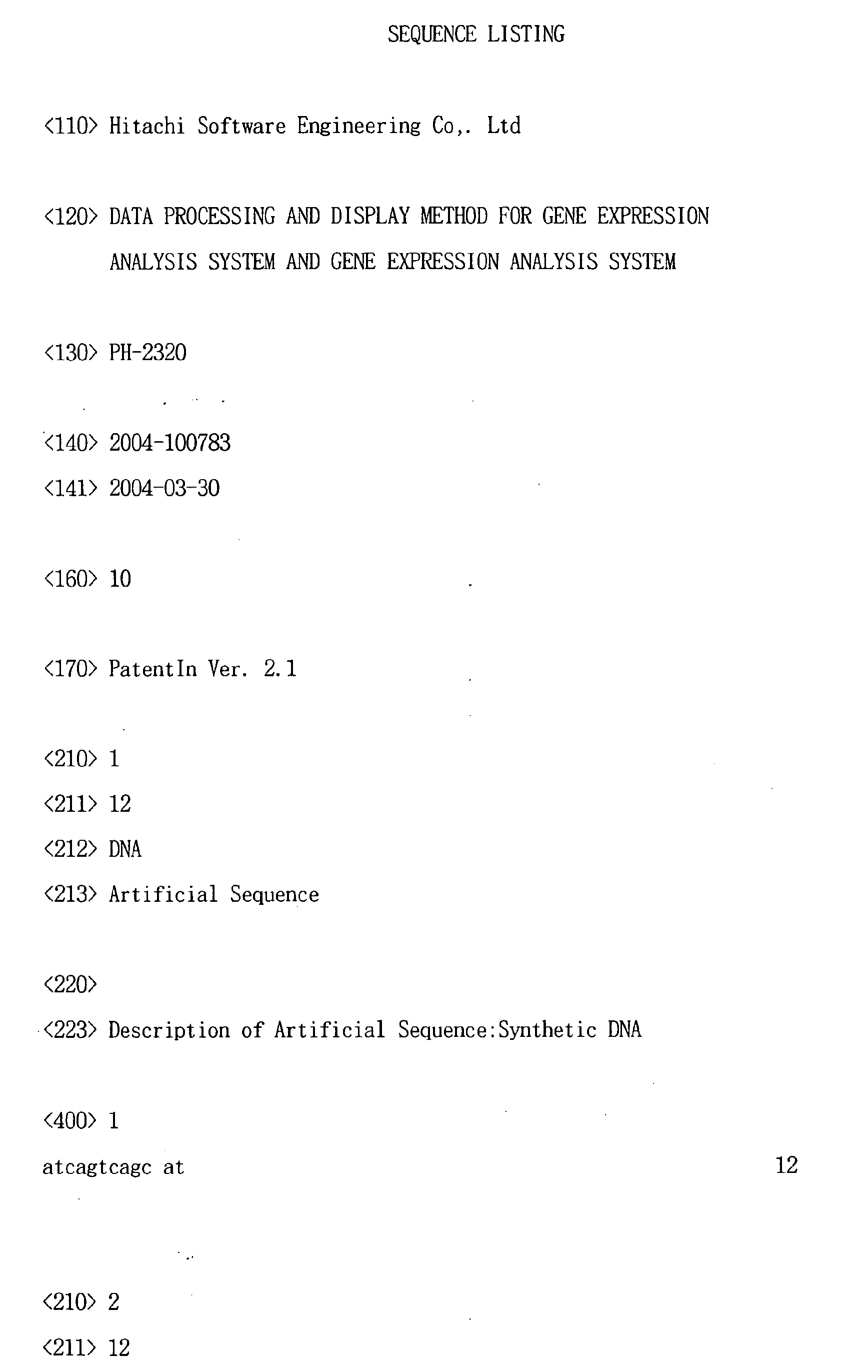

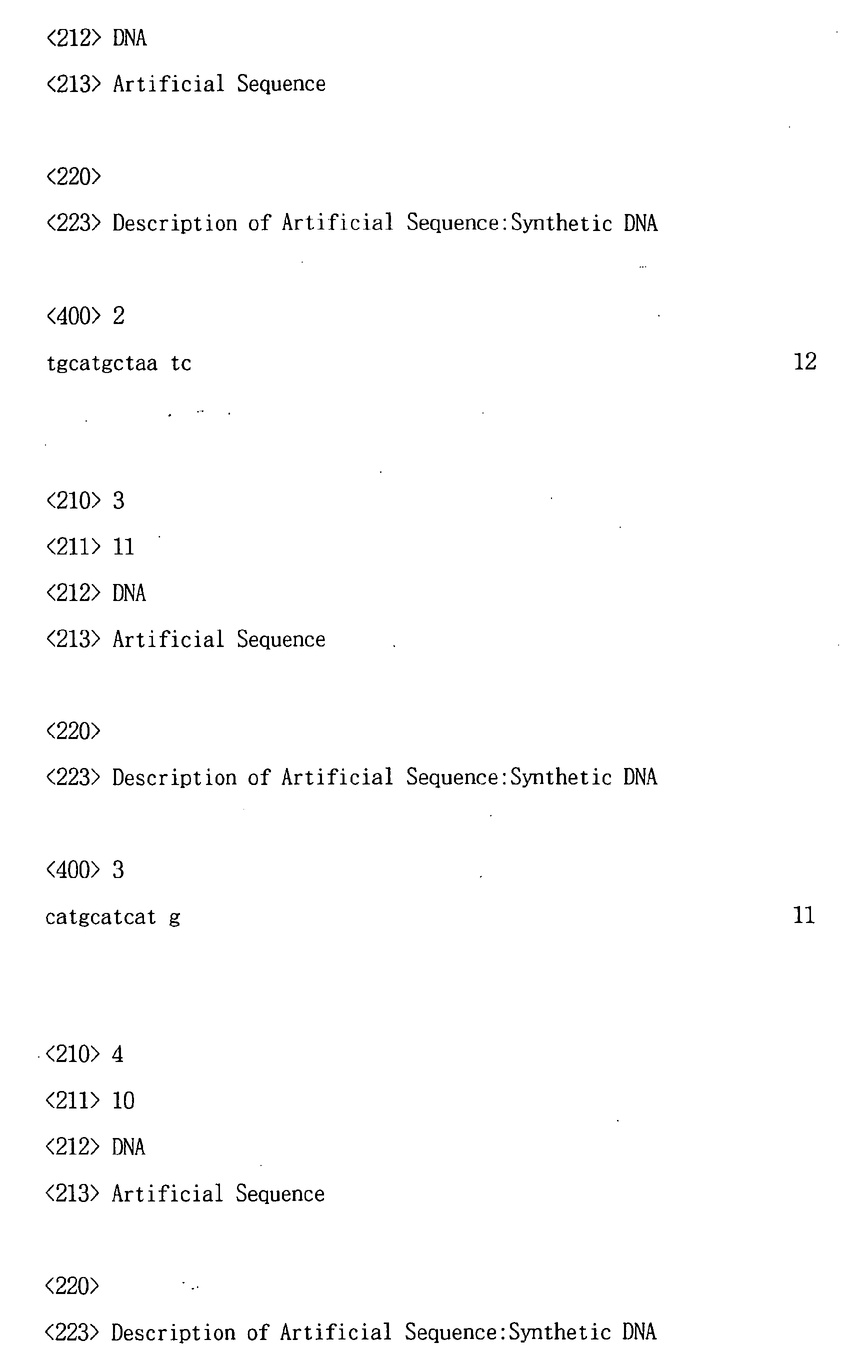

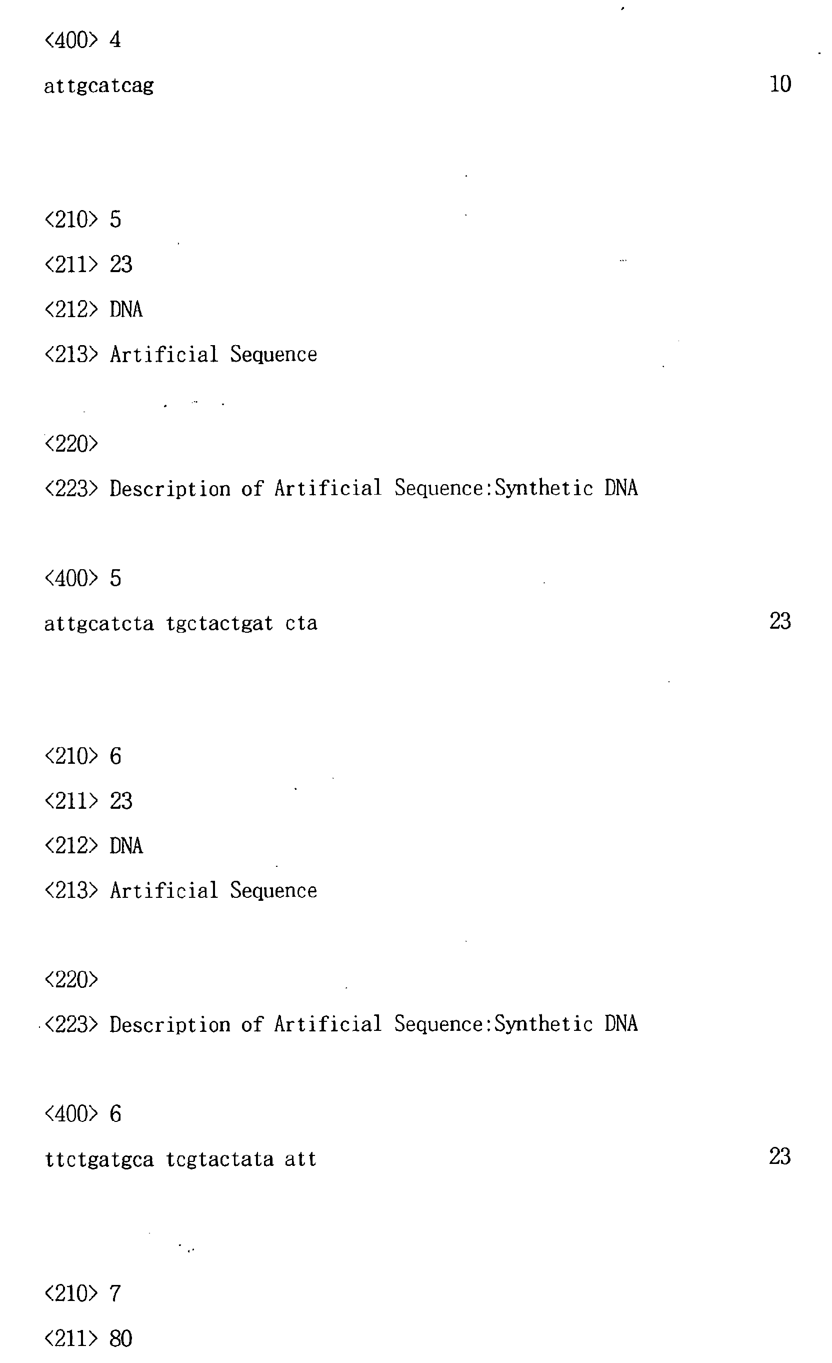

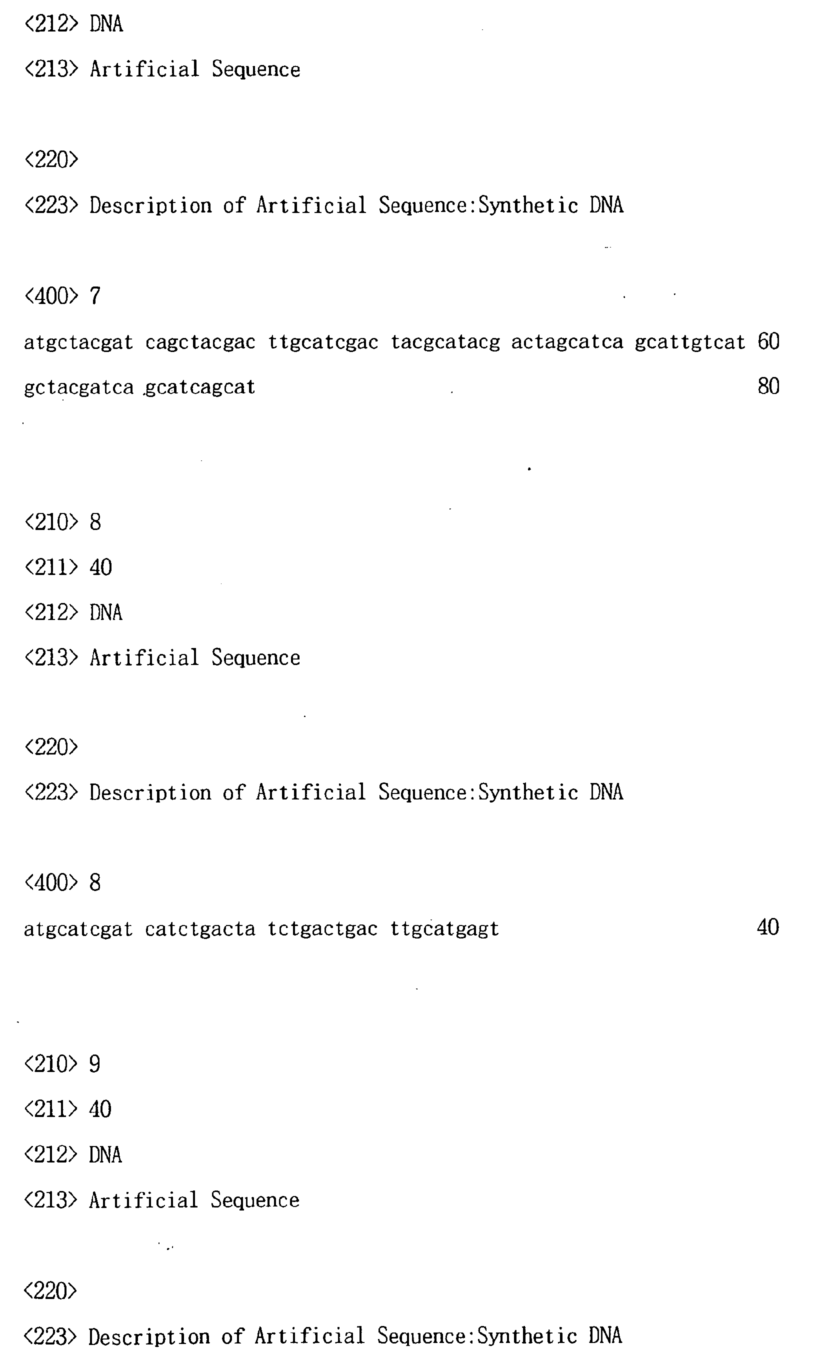

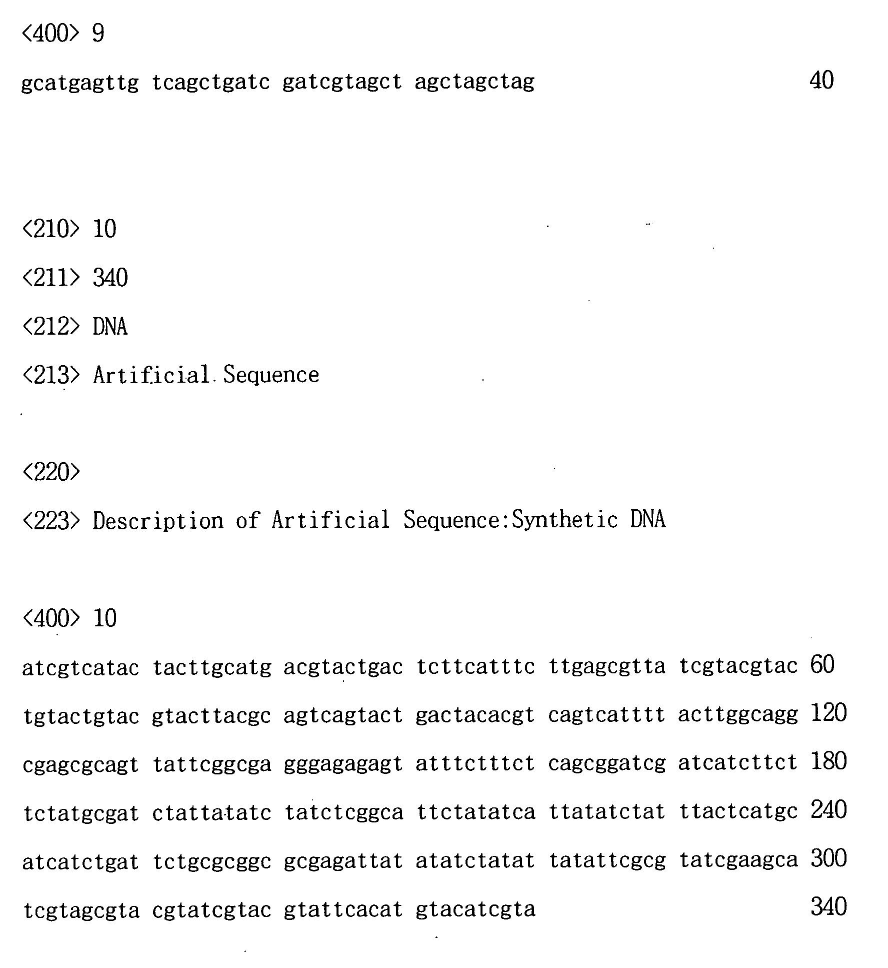

## Claims

1. A data processing and display method for gene expression analysis system, comprising the steps of:
inputting data of hybridization experiments carried out using a DNA chip provided with predetermined probe sequences; displaying graphically a specified length of a genome sequence including display of the position of any one of the probe sequences; and
displaying candidates for splice variants hybridizing to the probe sequence so as to correspond to the genome sequence.

2. The data processing and display method for gene expression analysis system according to claim 1, wherein the step of displaying the genome sequence includes displaying the specified length of the genome sequence in a strip shape and the position of the probe sequence on the genome sequence.

3. The data processing and display method for gene expression analysis system according to any of the preceding claims, wherein the step of displaying the genome sequence further includes displaying a chromosome number, an organism, position numbers of the start position and the end position of the genome sequence, position numbers corresponding to the start position and the end position of the probe sequence on the genome sequence, and an identification number of the probe sequence.

4. The data processing and display method for gene expression analysis system according to any of the preceding claims, wherein the step of displaying the genome sequence includes displaying expression values resulting from hybridization as numerals and in colors.

5. The data processing and display method for gene expression analysis system according to claim 4, wherein the step of displaying the genome sequence includes the step of determining an average expression value when there are a plurality of expression values resulting from hybridization and an average expression value for expression values of a plurality of different probes when said probes are located in the same position on the genome sequence.

6. The data processing and display method for gene expression analysis system according to any of the preceding claims, wherein the step of displaying candidates for splice variants includes displaying mRNA sequences and EST sequences of the candidates for splice variants and displaying intron sequences and exon sequences of the candidates for the splice variants.

7. The data processing and display method for gene expression analysis system according to any of the preceding claims, wherein the step of displaying candidates for splice variants includes compressing lengths of intron sequences for display such that the lengths of the intron sequences become relatively shorter than the lengths of exon sequences.

8. The data processing and display method for gene expression analysis system according to any of the preceding claims, wherein the step of displaying candidates for splice variants includes displaying link information that serves as information source concerning translation products of mRNA sequences and EST sequences of the candidates for the splice variants.

9. The data processing and display method for gene expression analysis system according to any of the preceding claims, further comprising the step of displaying a chromosome map including chromosomes with respective numbers of which lengths of long and short arms are graphically depicted so as to correspond to the genome sequence.

10. The data processing and display method for gene expression analysis system according to claim 9, wherein the step of displaying the chromosome map includes displaying expression values resulting from hybridization on the chromosomes in colors.

11. The data processing and display method for gene expression analysis system according to any of the preceding claims, further comprising the step of displaying experiment information that displays hybridization identification information to identify hybridization and experiment identification information to identify the hybridization experiments so as to correspond to the genome sequence.

12. The data processing and display method for gene expression analysis system according to claim 11, wherein the step of displaying the genome sequence includes displaying the genome sequence corresponding to the selected hybridization-identification information or experiment-identification information when a command to select one piece of the hybridization-identification information or one piece of the experiment-identification information displayed in the step of displaying experiment information; and the step of displaying candidates for splice variants includes displaying the candidates for the splice variants corresponding to the selected hybridization-identification information or experiment-identification information.

13. The data processing and display method for gene expression analysis system according to any of the preceding claims, wherein when a command to select an identification number of the probe sequence displayed in the step of displaying genome sequence is input, the identification number of the probe sequence, the probe sequence, an accession number, a gene name, and a description of the probe are displayed on a different window.

14. The data processing and display method for gene expression analysis system according to any of the preceding claims, wherein when a command to select mRNA sequence or EST sequence displayed in the step of displaying candidates for splice variants is input, basic information including an accession number, a definition of the mRNA or EST, a protein identification number, and a protein definition is displayed on a different window.

15. The data processing and display method for gene expression analysis system according to claim 14, wherein results of homology search performed based on the basic information are displayed.

16. The data processing and display method for gene expression analysis system according to any of the preceding claims, wherein when a command to select mRNA sequence or EST sequence displayed in the step of displaying experiment information is input, information on the DNA chip and hybridization is displayed so as to correspond to the genome sequence.

17. The data processing and display method for gene expression analysis system according to any of the preceding claims, wherein the step of displaying the genome sequence includes the step of searching for the position of the probe sequence to search an external database for information on the position of the probe sequence on the genome sequence.

18. The data processing and display method for gene expression analysis system according to claim 17, wherein the step of searching for the position of the probe sequence includes searching a local DNA chip database for information on the position of the probe sequence on the genome sequence and further the external database when the information is not stored in the DNA chip database.

19. The data processing and display method for gene expression analysis system according to claim 17, wherein the step of searching for the position of the probe sequence includes storing the information on the position of the probe sequence on the genome sequence extracted from searching the external database in the local DNA chip database.

20. The data processing and display method for gene expression analysis system according to claim 17, wherein the step of searching for the position of the probe sequence includes the step of extracting information on the position of the probe sequence from gene information (mRNA, EST) by searching the external database using annotation information of the probe as a search key and determining, using all sequences of probes on a DNA chip as query sequences and the genome sequence in the same position as that of the probe as a subject sequence, the positions of the probes on the genome sequence by performing multiple alignments.

21. The data processing and display method for gene expression analysis system according to claim 17, wherein the step of searching for the position of the probe sequence includes the step of performing a search by homology search program using a gene (mRNA) on a search key as a query sequence and all mRNA and EST sequences of an organism to be analyzed in the external database as subject sequences; and the step of performing a search by the homology search program using an obtained mRNA or EST as a query sequence and the genome sequence of the organism to be analyzed in the external database as a subject sequence.

22. The data processing and display method for gene expression analysis system according to any of the preceding claims, wherein the step of displaying candidates for splice variants includes the step of searching for splice variants to search an external database for the candidates for the splice variants.

23. The data processing and display method for gene expression analysis system according to claim 22, wherein the step of searching for splice variants includes storing information on the candidates for the splice variants extracted from searching the external database in a local DNA chip database.

24. The data processing and display method for gene expression analysis system according to claim 22, wherein the step of searching for splice variants includes searching for the candidates for the splice variants using homology search program.

25. The data processing and display method for gene expression analysis system according to claim 22, wherein the step of searching for splice variants includes the step of executing a program with homology search program between base sequences according to conditions of predetermined parameters using the entire database sequences to be searched as subject sequences; and the step of storing, in a DNA chip database, results obtained from determining the position of a query sequence with respect to a subject sequence, determining the position of the subject sequence with respect to the query sequence, and selecting calculated homology search outcomes.

26. The data processing and display method for gene expression analysis system according to claim 9, wherein the step of displaying a chromosome map includes the step of searching for chromosomes to search an external database for information on the chromosomes.

27. The data processing and display method for gene expression analysis system according to claim 26, wherein the step of searching for chromosomes includes storing the information on the chromosomes extracted from searching the external database in a local DNA chip database.

28. The data processing and display method for gene expression analysis system according to any of the preceding claims, further comprising the step of preprocessing and normalization to perform preprocessing and normalization of the data; and the step of statistical analysis to analyze the data statistically.

29. A gene expression analysis system comprising:
a data input unit that inputs data concerning hybridization experiments carried out using DNA chips with predetermined probe sequences;
a preprocessing and normalization unit that performs preprocessing and normalization of the data;
a genome sequence display processing unit that generates data for displaying graphically a specified length of a genome sequence that includes displaying the position of any one of the probe sequences; and
a display unit for candidates for splice variants that generates data to display the candidates for splice variants hybridizing to any one of the probe sequences so as to correspond to the genome sequence;
wherein the step of statistical analysis to analyze the data statistically is provided.
